(19) Europäisches Patentamt

European Patent Office

Office européen des brevets

(11) **EP 2 579 766 B1**

(12) **FASCICULE DE BREVET EUROPEEN**

(45) Date de publication et mention de la délivrance du brevet:
**21.12.2016 Bulletin 2016/51**

(21) Numéro de dépôt: **11735457.1**

(22) Date de dépôt: **08.06.2011**

(51) Int Cl.:
*A61B 1/24* *(2006.01)*      *A61B 5/107* *(2006.01)*
*A61B 5/00* *(2006.01)*      *A61B 1/00* *(2006.01)*
*A61B 1/06* *(2006.01)*      *A61B 1/247* *(2006.01)*

(86) Numéro de dépôt international:
**PCT/FR2011/051300**

(87) Numéro de publication internationale:
**WO 2011/154656 (15.12.2011 Gazette 2011/50)**

(54) **DISPOSITIF DE MESURES TRIDIMENSIONNELLES ET TEMPORELLES PAR EMPREINTE OPTIQUE EN COULEUR**

VORRICHTUNG ZUR AUFNAHME DREIDIMENSIONALER UND ZEITLICHER OPTISCHER ABDRÜCKE IN FARBE

DEVICE FOR TAKING THREE-DIMENSIONAL AND TEMPORAL OPTICAL IMPRINTS IN COLOUR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.06.2010  FR 1054483**

(43) Date de publication de la demande:
**17.04.2013  Bulletin 2013/16**

(73) Titulaire: **Duret, François**
**11560 Fleury d'Aude (FR)**

(72) Inventeur: **Duret, François**
**11560 Fleury d'Aude (FR)**

(74) Mandataire: **Rhein, Alain**
**Cabinet BREV&SUD**
**55 Avenue Clément Ader**
**34170 Castelnau le Lez (FR)**

(56) Documents cités:
**EP-A1- 2 166 303          US-A1- 2009 227 875**
**US-A1- 2009 259 098**

**Description**

La présente invention a pour objet un nouveau dispositif de mesures tridimensionnelles et temporelles par empreinte optique en couleur d'un volume de quelques centimètres cubes à la surface du corps humain, assurant son intégrité structurale, applicable dans le domaine dentaire pour les prises de vues endobuccales, mais aussi assurant dans ce domaines, une aide au diagnostic comportant, un système stéréoscopique miniaturisé associé à un ou plusieurs capteurs électroniques de type CCD ou Cmos couleur un éclairage spécifique et modulé à LEDs de une ou plusieurs longueurs d'onde permettant de mesurer des surfaces régulières spéculaires ou lambertiennes sans dépôt de "coating" à la surface des dents ou de la gencive, une unité centrale de gestion et de conversion des données analogiques digitales, mais aussi et éventuellement des logiciels d'analyse de teintes et mouvements pour des aides aux diagnostics par une réflexion, pénétration globale ou sélective des rayonnements lumineux LED judicieusement sélectionnés dans l'éclairage utilisé, sans nécessité le moindre balayage mécanique, optique ou électro optique.

La prise d'empreinte par des moyens optiques en vue de réaliser des diagnostics ou des prothèses a été décrite pour la première fois en 1973 par le demandeur dans sa thèse de deuxième cycle (DDS) sous le titre "Empreinte optique". Le demandeur a fait de nombreuses publications sur ce sujet. Il a notamment déposé le premier brevet traitant de l'interférence pour les prises d'empreintes optiques endobuccales dans les documents US 4.663.720 et US 4.742.464, et également US 4.611.288, mais aussi US 5.092.022. Le demandeur a également proposer la prise d'empreinte optique en dentisterie et médecine par projection de masques, FR 84.05173), par balayage en phase profiliométrique en projection conique US 4.952.149, ou par le suivi dynamique par LEDs WO 94/00074.

Dès 1982 de nombreux documents traitent de la prise d'empreinte optique par balayage en phase profiliométrique en projection parallèle, de la modélisation ou de l'usinage de la prothèse comme.

Tous ces travaux et inventions ont conduit à de nombreuses réalisations et plus de vingt systèmes commercialement disponibles.

Depuis 2000, il est proposé des solutions différentes, non pas en bouche, mais sur des modèles en plâtre réalisés à partir d'empreintes faites en bouche par des méthodes traditionnelles, par exemple dans le document US 7.399.181, ou sur des modèles construits par stéréolythographie, document US 10.726.257. Cette solution a été aussi proposée en complément des systèmes pour dentistes avec un scannage sur modèle par projection de points ou de trames, document US 7.335.876.

Dans le domaine de l'orthodontie d'autres propositions ont été faites pour utiliser l'empreinte optique comme le montre le document US 7.361.018. Ces systèmes ont permis, entre autre, le développement commercial du système décrit dans les documents US 7.361.017, US 7.393.208, ou US 6.318.994, US 6.802.713, US 11.405.972.

Comme on peut le voir, dans tous ces systèmes, peu sont transposables en bouche pour les raisons suivantes:

- le scannage est trop lent, le balayage passe de 2 minutes par dent à 2 secondes pour les plus rapides,

- l'appareil oblige à avoir une caméra en position constante par rapport à l'objet, ce qui obligerait à fixer la caméra, et la tête du patient,

- le mécanisme de déplacement reste complexe et imprécis.

En plus de ces inconvénients, tous les systèmes dits de laboratoire, scannant un modèle, conduisent le dentiste à réaliser une empreinte traditionnelle, ce qui n'élimine pas la gêne du patient et l'imprécision du moulage endobuccal et oblige le praticien à envoyer la pièce au laboratoire. En plus de cet inconvénient, le prothésiste, en coulant l'empreinte rajoutera d'autres erreurs nuisant considérablement à la précision de l'empreinte optique sur laquelle il travaillera avec son logiciel de conception assistée par ordinateur (CFAO) après scannage.

Aujourd'hui les systèmes travaillant en bouche sont actuellement peu nombreux. Tous ces systèmes utilisent le balayage mécanique, optique ou électro optique pour effectuer la mesure de la surface étudiée. Ces méthodes peuvent être classées selon trois types, l'une utilisant la profilométrie de phases en projection parallèle en lumière visible ou bleue, à projection conique, par balayage de franges rouges ou infra rouges en une centaine de milli secondes et enfin, récemment le système décrit dans le document US 7.372.642.

Pourtant toutes ces caméras endobuccales, y compris celle développée par le présent demandeur, présentent plusieurs inconvénients particulièrement rédhibitoires:

- ces systèmes sont complexes à mettre en oeuvre et exigent beaucoup de soins de calibrage.

- l'électronique reste complexe ce qui rend difficile toute diminution de prix et rend fragile la caméra.

- le coût de la caméra est particulièrement élevé et peu dépasser les 30.000 €.

- les caméras sont en général volumineuses et lourdes, ce qui gène l'utilisateur.

En réalité, une analyse plus fine montre que ces caméras présentent plusieurs inconvénients très importants, dans le principe même des méthodes utilisées. Ces inconvénients sont incontournables car ils sont liés au choix de ces méthodes.

a) Tous ces systèmes, que ce soit en bouche, sur la peau ou en laboratoire (sur modèle), qu'ils utilisent l'OCT (Optical Coherence Tomography) en dermatologie ou l'ophtalmologie, utilisent le balayage de la surface par des moyens mécaniques, optiques ou électro optiques. Même si ce balayage de franges ou de trames est très rapide, il n'en reste pas moins qu'il oblige à un mouvement dans la caméra elle-même, mouvement qui peut entraîner des flous ou des déplacements parasites conduisant souvent au rejet d'une partie des vues.

b) ce balayage limite significativement la profondeur de champ déjà considérablement réduite dans une vue macroscopique (de quelques cm cubes).

c) ce ne sont pas les points de la surface de l'objet qui sont mesurés mais la déformation d'une projection lumineuse à la surface de cet objet. Cette première caractéristique oblige les développeurs à recouvrir les dents d'une couche blanche dite de "coating", dégradant par principe, la mesure réelle de l'objet. Ceci est d'ailleurs souvent exprimé à la fois comme une imprécision et une gêne dans l'utilisation des caméras en bouche.

d) ceci a obligé les fabricants à utiliser des rayonnements rendant la dent "opaque" comme le font les rayons bleus ou les ultra violets. C'est la raison pour laquelle le présent demandeur avait proposé un appareil utilisant un laser Argon. Ceci peut être contraignant pour l'utilisateur, voire dangereux, pour le patient.

e) plus encore, le fait de ne pas mesurer l'objet, mais la déformation de la lumière projetée, que ce soit un point, une ligne, une trame ou une phase de cette lumière, supprime toutes possibilités d'avoir une correspondance parfaite entre la couleur, la teinte de l'objet et sa mesure. La seule teinte que l'on puisse avoir est la couleur de la lumière projetée.

f) le passage de la lecture 3D à la lecture 2D en couleur, si on l'utilise pour les diagnostics, est totalement impossible en dentisterie car c'est seulement une image monochromatique représentant la lumière des franges qui est récupérée.

g) enfin les techniques d'analyse par profilométrie ou balayage obligent à la prise de plusieurs vues du même endroit pour que l'on puisse extraire la troisième dimension. Cela se traduit pas un risque de déformation des données entre la première vue et la dernière conduisant à de grosses erreurs de corrélation et de précision. Le "bougé" a toujours été l'ennemi de ce type de technologie.

En fin de compte s'il est possible de mesurer une dent, c'est toujours la mesure de la lumière projetée qui est réalisée et non pas l'objet lui-même et cette mesure oblige à l'utilisation de mouvements de la source ou des optiques durant la lecture. Comme dit précédemment, tous ces systèmes sont basés sur la mesure de la déformation de la lumière déplacée et visualisée par la caméra.

On notera qu'il en est de même dans le domaine de la dermatologie ou l'ophtalmologie. Les méthodes utilisées en lecture 3D sont récentes coûteuses et complexes comme le montrent les appareils en OCT. C'est pourquoi ces disciplines font surtout appel à des mesures 2D moins lourdes pour les études sous cutanées anatomiques ou leurs expansions à la pathologie (éventuelle).

Les techniques utilisées aujourd'hui sont les suivantes :

a) le vidéodermatoscope qui consiste en un outil actuellement très utilisé, basique, permettant d'avoir une image magnifiée de la peau (jusqu'à 70x). La technologie digitale permet de prendre des photographies numériques ainsi que des enregistrements, facilitant ainsi la comparaison dans le temps et le partage d'informations entre cliniciens. Les appareils offrent d'autre part des fonctionnalités annexes, comme la possibilité d'utiliser des sources lumineuses de différentes longueurs d'onde pour éclairer la peau, ou encore des traitements de l'image tels que la segmentation automatique des lésions ou encore l'extraction automatique des critères ABCD.
Le coût d'un tel appareil reste toutefois élevé, et aucune étude clinique cherchant à montrer une amélioration dans le diagnostic par rapport à l'examen clinique simple n'a été trouvée. De plus le vidéodermatoscope ne fournit pas d'information en profondeur.

b) l'échographie, qui permet l'exploration en profondeur des lésions. Grâce à des fréquences de l'ordre de 10 - 50 MHz, il est possible de descendre jusqu'à 12mm avec une résolution axiale de 150 μm. Cette technique est utilisée pour l'étude de l'extension sous-cutanée en analyse préopératoire et la recherche de mélanomes métastatiques, où elle a montré d'excellentes capacités en terme de sensibilité et de spécificité. Cependant, la bonne utilisation d'un tel appareil nécessite toutefois d'acquérir une certaine expérience dans la lecture d'image d'échographie ; d'autre part, il est beaucoup plus difficile d'ajouter des post traitements informatifs sur ces images, contrairement aux techniques multispectrales (cf infra).

c) l'OCT, qui est basée sur des techniques optiques interférométriques, permettant d'imager la peau en profondeur en 3D avec une bonne résolution latérale (de l'ordre de 15 μm), supérieure à celle des échographes. Il permet d'autre part de faire de l'imagerie en quasi temps réel, mais est limitée en profondeur (maximum 1,5 - 2mm). Un seul appareil est actuellement commercialisé, et l'étude de son efficacité dans le diagnostic du mélanome est actuellement en cours d'étude. S'il a une très bonne résolution en imagerie temps réel il travaille sur une faible profondeur, n'a pas de données cliniques, travaille en coupe, est difficile à mettre en oeuvre et est très cher.

d) la microscopie confocale, qui fournit des images 3D de l'épiderme et du derme papillaire avec une très haute résolution (résolution latérale 1-2 pm, résolution axiale 3-5 pm). Son principal inconvénient est qu'elle est très limitée en profondeur (200-500 pm).
Ces appareils ont l'avantage d'avoir une excellente résolution, une très bonne discrimination mélanome / naevus (meilleure que l'examen clinique seul). Mais, en dehors d'être d'un coût très élevé, ils ont une très faible profondeur d'analyse.

e) l'Imagerie multispectrale, qui consiste en la technique suscitant le plus d'intérêt aujourd'hui de par la simplicité de la méthode et de son bon rapport qualité / prix. C'est en effet une technique d'imagerie simple : elle suppose que la peau est organisée en couches, et que chaque couche comporte des proportions différentes de substances appelées chromophores qui ont chacune un spectre d'absorption de la lumière relativement caractéristique. Les principaux chromophores de la peau étant la mélanine, le collagène et l'hémoglobine, on comprend tout l'intérêt de cette méthode dans l'étude du mélanome, où la proportion de mélanine va être modifié sur un nombre plus ou moins important de couches. Pour obtenir des informations spatiales quantitatives sur ces chromophores, on projette sur la peau différentes lumières monochromatiques (classiquement dix), et on mesure la lumière réémise par le tissu cutané pour chaque longueur d'onde. On obtient donc des informations en profondeur sur lesquelles on peut appliquer des traitements automatiques, notamment segmenter les lésions, en obtenir les critères ABCD en profondeur et en quantifier la proportion en chromophores. Toutefois, on ne peut atteindre des profondeurs que de l'ordre de 2.5mm. Les principaux avantages des appareils sont leur technique simple à mettre en oeuvre, les nombreux traitements automatiques possibles et leur bonne discrimination mélanome / naevus (meilleure que l'examen clinique seul). Ils ont les inconvénients de ne travailler qu'en 2D, d'être encore couteux, et de travailler avec une profondeur assez limitée.

Certes il existe des méthodes à l'étude pour les pathologies de la peau utilisant les principes de l'IRM, du PET - scan, de l'imagerie bi-photonique ou de l'imagerie terahertz mais leur mise en oeuvre sera longue et elle conduira à des appareils trop coûteux pour être utilisables en cabinet privé, ce qui reste l'objectif à atteindre.
Enfin, il y a eu quelques essais de mesures stéréographiques en médecine, utilisant deux ou plus de deux capteurs, qui par méthode de triangulation, permettent de remonter à la troisième dimension. L'utilisation de deux capteurs permet une vision stéréoscopique dans les objets parfaitement définis mais les méthodes de corrélations mathématiques sont complexes et coûteuses car les objets servant de repères sont difficilement identifiables. L'action manuelle est pratiquement toujours nécessaire et les essais effectués sur des dents se sont avérés inexploitables dans les distances et profondeurs de champ visés.
De même, le développement des images dites en "triplet imaging système" (L configuration), utilisant des caméras placées dans une position de triangulation équilatérale, a apporté des informations intéressantes dans la détermination de la troisième dimension en simplifiant la triangulation, mais les résultats se sont avérés inexploitables dans les conditions dentaires exposées ci-dessus. En effet tous les systèmes utilisés obligent à connaître le déplacement de la caméra ou de l'objet entre deux (ou n) acquisitions.
Tous ces inconvénients ont conduit à proposer une solution universelle peu coûteuse répondant aux critiques faites plus haut.
En outre, on connait également la demande de brevet US 2009/0227875 qui divulgue un dispositif de prise de vue adapté à prendre en trois dimensions des empreintes optiques d'un objet à étudier dans le domaine dentaire.
US 2009/259098D1 divulgue un endoscope médical non adapté au domaine dentaire. Cet endoscope comprend un capteur d'image et une lentille de focalisation déformable.

La demande de brevet US 2008/045789 divulgue une capsule endoscopique qui n'est pas adaptée au domaine dentaire et qui comprend des mécanismes pour ajuster la distance focale et le zoom.

Enfin, la demande de brevet EP 2 166 303 divulgue un dispositif qui est destiné à la détermination de coordonnées 3D d'une dent et qui utilise à cet effet la projection d'une lumière structurée.

La présente invention a pour but de résoudre les inconvénients précités en proposant un ensemble complet de lecture endobuccale associant lecture 3D dynamique très rapide voire instantanée, visualisation en couleur, analyse du proche sous-cutané et possibilité de basculage, en temps réel, en visualisation 2D, le tout conduisant à une numérisation très précise sans adjonction de "coating".

Ainsi, le dispositif de mesures tridimensionnelles et temporelles par empreinte optique en couleur utilisable dans le domaine dentaire selon l'invention se caractérise essentiellement en ce qu'il consiste en un dispositif de prise de vue dentaire en trois dimensions n'util isant pas de projection de lumière structurée, et comportant à cet effet:

- une caméra stéréoscopique composée d'au moins deux capteurs en position prédéfinie, de type CCD ou Cmos couleurs définissant, par leur vitesse de rafraîchissement, la vitesse de lecture donc la vitesse des prises d'empreintes successives, et permettant une lecture statique ou dynamique,

- un système optique, à focale fixe et prédéfinie, permettant de transmettre auxditx capteurs, sans déformation, les données visualisées sur le champ opératoire,

- un système d'éclairage à LED, destiné à éclairer la zone de prise d'empreinte,

- un système électronique situé derrière ou à proximité de chacun desdits capteurs, assurant la gestion de celui-ci, mais aussi celle des LEDs éclairant la zone de prise d'empreinte,

et en ce que ledit système électronique comporte:

- une unité centrale de gestion apte à collecter, stocker et ordonner les données relevées par lesdits capteurs,

- une carte de contrôle desdites LEDs, sous la dépendance de ladite unité centrale,

et en ce que lesdits capteurs sont répartis sur toute ou partie d'une arcade dentaire afin de réaliser une empreinte en un seul cliché, évitant le balayage clinique de l'arcade du patient par l'opérateur, ils sont disposés dans une sorte de porte-empreinte optique permettant la saisie complète de l'arcade en un seul cliché.

Le système optique peut être un endoscope, si les capteurs sont placés hors de la zone de lecture, ou un simple système de lentilles si les capteurs sont placés contre la zone de lecture. Le système peut donc être très simple, ce qui est totalement impossible dans les techniques dites à lumière structurée où il est nécessaire de disposer d'un conduit pour la lumière projetée et un autre pour l'image réfléchie.

L'unité centrale de gestion est également apte, éventuellement, à convertir les données de valeurs analogiques en valeurs numériques. Le fait de ne pas devoir gérer un système de projection de masques ou de franges réduit significativement l'unité centrale à son strict minimum: la gestion d'une caméra double vue stéréoscopique couleur.

La carte de contrôle est apte à déclencher préférentiellement telle ou telle LED en fonction des programmes mis en oeuvre. En effet les LEDs seront commandées alternativement ou ensemble, ou en ordre variable en fonction du programme mis en oeuvre.

Le système électronique comporte également :

- une carte alimentation de type standard capable de fonctionner sous USB ou sous batterie. Suivant qu'on utilise un système libre (sans connexion par fil) ou connecté, l'alimentation reste légère compte tenu de la faible consommation des composants mis en oeuvre. La caméra est donc la première à pouvoir disposer d'une connexion sans fil,

- une carte mémoire miniaturisée inclue dans la caméra, permettant de stocker les vues et de les transférer à l'ordinateur à l'aide d'un support transportable sans avoir besoin de connexion USB,

Le système d'éclairage est un système d'éclairage à LEDs de différentes longueurs d'onde ou de couleurs dont la mixité peut conduire, par exemple, à la lumière blanche ou "de jour" afin d'avoir une visualisation couleur réelle ou stimulée (fluorescence). Le choix judicieux des LEDs permettra:

- soit de visualiser la zone mesurée en lumière du jour (LED dite "blanche")

- soit, par activation des LEDs, de mettre en évidence certains tissus comme, les tissus minéralisés dans le bleu ou les UV, un tissu dentaire fluorescent donc d'aspect particulièrement "mat"

- soit, encore, de visualiser certaines pathologies "dermiques" en fonction des longueurs d'onde sélectionnées. En particulier on sait que les pénétrations des longueurs d'onde peuvent être corrélées avec certaines pathologies, le choix judicieux du balayage de la zone permettra de visualiser ce qui n'est pas visible à l'oeil, et ceci en relief.

- soit de permettre de repérer des points de calage de corrélation caractérisés par la couleur de marquage utilisé à la surface mesuré.

Cette même application permet aussi de pénétrer des zones de gencive plutôt fines, comme on le sait dans le sulcus dentaire, offrant à l'opérateur une vue de l'émergence de la dent. De même une couleur, par exemple dans les rouges, permet de s'affranchir des effets salivaires néfastes, à la différence des méthodes actuelles.

Le dispositif comporte également un ordinateur de type standard, portable, embarqué ou de bureau, renfermant un logiciel de gestion et de traitement des programmes et des données, capable de restituer les informations sous une forme visible à l'écran, 2D ou 3D, mais aussi d'adresser les mesures à des centres plus ou moins éloignés (internet, Wifi, ethernet ...) sous une forme standard assimilable à tout système de CFAO (STL..) ou sous une forme spécifique, moyennant des logiciels de traduction de langage. C'est dans cet ordinateur, avant de disposer d'unité de calcul miniaturisé, que se placera le logiciel de restitution 3D et de commande de la caméra.

La présente invention concerne ainsi un dispositif de lecture stéréoscopique couleur, miniaturisé et dynamique d'une petite partie du corps humain associé à une caméra de n CCD ou n Cmos, à un système de conversion analogique digitale, à une unité centrale de gestion des données, à une sortie des informations sous forme standard ou spécifique, à une amplification du message reçu par utilisation de LEDs de couleur blanche ou de valeurs spectrales spécialement désignées, et une connexion ondulatoire (par exemple Wifi) ou par port USB auto alimenté, et par une alimentation par batterie ou par connexion électrique.

Cette invention permet de répondre aux problèmes exposés ci-dessus, en proposant une solution modulable, peu onéreuse et utilisable dans tous les cabinets dentaires mais aussi comme instrument à main dans les laboratoires de prothèse, sous une forme simplifiée et agréable pour le patient.

En particulier cette invention répond aux nombreux problèmes évoqués précédemment :

1) par les moyens mis en oeuvre, le dispositif est simple dans sa fabrication ce qui le rend particulièrement résistant,

2) cette simplicité a une conséquence essentielle sur le prix de fabrication, donc sur le prix de vente particulièrement depuis la démocratisation des éléments électroniques utilisés comme les CCD, les Cmos ou les LEDs,

3) l'alimentation, particulièrement simple, peut être contenue dans la liaison USB de connexion avec tous types d'ordinateurs capables de recevoir le langage standard de sortie type, par exemple STL,

4) la manipulation d'images stéréoscopiques, base de cette nouvelle invention permet un auto calibrage supprimant tout ajustage dans le temps, ce qui n'est pas le cas des méthodes actuelles utilisant les lumières dites structurées,

5) le fait de travailler avec des capteurs CCD ou Cmos dans une position spatiale prédéfinie, immuable et figée durant la fabrication, les uns par rapport aux autres, évite la nécessité de connaître les mouvements de l'objet ou des caméras (les uns par rapport aux autres), ramenant le problème de disparité à un simple problème de corrélation de densité dans le nuage de points,

6) le fait de ne pas utiliser de mesures de déformation de lumière structurée permet de travailler sur des images du corps lui-même en couleur,

7) le fait d'avoir des images en couleur permet de sélectionner sélectivement manuellement ou automatiquement certaines parties du corps humain, par exemple de reconnaître les dents et la gencive séparément,

8) le fait de travailler sans lumière structurée permet aussi de ne pas avoir recours à des surfaces de "coating", facteur d'imprécision notoire lorsque l'on voisine des valeurs proches du micron, ce qui est nécessaire dans toutes mesures ayant pour objet l'ajustage d'une prothèse ou d'un diagnostic,

9) l'utilisation de diode électro luminescente permet de visualiser certaines zones légèrement sous-cutanées ou sous-gingivales afin de parfaire la lecture dans des zones invisibles sans avoir recours à des méthodes chirurgicales (rétractions gingivales) ou des méthodes plus complexes (OCT) pour des diagnostics simples,

10) elle permet aussi, par des marquages volontaires simples en couleur, de repérer plus facilement certaines zones naturellement colorées sur lesquelles on peut s'appuyer, ou de faciliter les corrélations de vues en sélectionnant l'éclairage d'une LED de couleur complémentaire au marquage,

11) l'auto corrélation étant aujourd'hui, avec les outils softwares récents, facilitée par les informations couleurs, permet de filmer en dynamique : une surface complexe (arcade entière), les mouvements de ces surfaces (arcades du haut par rapport aux arcades du bas),

12) l'utilisation d'un seul des capteurs d'images permet de basculer d'une image 3D en image 2D pour disposer, avec le même outil, d'une analyse planaire et d'une analyse spatiale, ce qui est la base de beaucoup d'appareils aujourd'hui sur le marché,

13) une seule vue doublée ou triplée au même moment, est suffisante pour extraire la troisième dimension, ce qui évite tout "bougé" dans le captage des données,

14) la restitution 3D à l'écran est rendue possible sur des écrans haute définition 3D standards ce qui n'est pas la cas, sans traitement complexe des systèmes endobuccaux actuels,

15) la simplicité des traitements permet l'utilisation d'un ordinateur standard de bas de gamme,

16) enfin, l'appareil proposé est universel dans son champ d'application, et répond à de nombreuses demandes en matière de coût, de précision et d'imagerie diagnostic.

Le dispositif selon l'invention permet d'avoir une vue dynamique en se déplaçant sur la zone d'analyse, à la différence des systèmes en phase profilométrique qui doivent faire un minium de quatre vues pour extraire le relief, le système utilisé dans la présente invention, se contente d'un seul cliché double, évitant tout bougé dans la mesure ou l'intégration de l'information sur le capteur est immédiate.

La présente invention consiste donc à réaliser une empreinte dentaire à l'aide d'un ensemble de capteurs CCD ou Cmos en position prédéterminée dans le montage géométrique du système optique associé à l'électronique de gestion des données hardwares (cartes de gestion) et softwares (logiciel de gestion des données) afin de résoudre le problème inhérent aux systèmes stéréoscopiques classiques (connaissance de la position variable des caméras les unes par rapport aux autres).

La présente invention consiste aussi à associer, de manière obligatoire, un système d'éclairage à LED de longueur d'onde prédéfinie, afin de permettre une mise en corrélation des vues statiques ou dynamiques de manière particulièrement facile au niveau de la reconnaissance des points de référence et des points de corrélation.

Selon un premier mode de réalisation, le dispositif comporte trois capteurs destinés à être régulièrement positionnés, selon une géométrie connue, autour de l'objet à étudier, des lentilles de focalisation fixes placées en face de chaque capteur suivant un axe optique central dont on connaît parfaitement la position et l'orientation spatiale des uns par rapport aux autres, lesdites lentilles, en position colinéaire par rapport à l'axe de vision du capteur, formant trois chemins d'images propres à chaque capteur.

Afin de simplifier la corrélation des vues et d'ajuster l'effet zoom entre chaque capteur, les captures d'images sont faites au même moment, ce qui signifie que les trois images 2D capturées, à angulations différentes, arrivent dans le système de traitement d'images au même moment. Il n'existe aucun "time out" entre chacune de ces prises de vues, ce qui a l'avantage de simplifier considérablement le traitement de l'image et la recherche de la troisième dimension. Cela a l'avantage de recaler les nuages de points apparus sur une zone vue par plusieurs capteurs et de corriger l'effet zoom apparaissant obligatoirement du fait de la position variable de l'objet par rapport à l'ensemble des capteurs.

Le calcul de la troisième dimension étant basé sur la recherche de points similaires, facilitée par le positionnement prédéfini des capteurs mais particulièrement difficile pour des surfaces uniformes comme le sont les surfaces dentaires, est associé à ces trois capteurs des LEDs dans le blanc pour révéler la couleur réelle de l'objet mesuré. Les points images renferment donc deux informations complémentaires, le temps (moment de la prise de vue qui deviendra commun à l'ensemble des trois vues pour simplifier les calculs), et, sans que cela soit obligatoire, une cinquième dimension elle-même divisible dans un système chromatique, et qui correspond à l'information couleur.

Avantageusement ces LEDs peuvent aussi être d'une longueur d'onde (de quelques nanomètres) prédéfinie permettant de mettre en évidence des marquages naturels (fonds de sillons ou zones de couleurs différenciant tumeurs, gencives ou teintes des dents) ou des sur marquages réalisés avant la prise d'empreinte, faits à l'aide de marqueurs de couleur complémentaire.

Avantageusement ces marquages peuvent être des objets de différentes formes, placés (dans la zone mesurée), collés ou logés (dans les espaces inter dentaires ou sur les têtes d'implant) sur l'objet analysé.

Avantageusement, cette ou ces LEDs sont placées autour de chaque lentille de focalisation de l'image placée devant le capteur.

Avantageusement ces LEDs peuvent être une association entre les LEDs blanches et de longueur d'onde prédéfinie afin que les mesures soient réalisées à partir de couleurs naturelles, et non artificielles.

Avantageusement, pour faciliter la recherche de la troisième dimension, et pour ramener les calculs à une géométrie à une dimension, préférentiellement la présente invention utilise des capteurs en position équilatérale.

Selon un deuxième mode de réalisation, le dispositif selon l'invention comporte deux capteurs régulièrement positionnés autour de l'objet étudié selon une géométrie connue. Les lentilles de focalisation sont placées en face de chaque capteur suivant un axe optique central dont on connaît parfaitement la position et l'orientation spatiale des uns par rapport aux autres. Ces lentilles, en position colinéaire par rapport à l'axe de vision du capteur forment deux chemins d'images propres à chaque capteur.

Afin de simplifier la corrélation des vues et d'ajuster l'effet zoom entre chaque capteur, les captures d'image sont faites au même moment, ce qui signifie que les deux images 2D capturées, à angulation différente, arrivent dans le système de traitement d'images au même moment. Il n'existe aucun "time out" entre chacune de ses prises de vues, ce qui aura l'avantage de simplifier considérablement le traitement de l'image et la recherche de la troisième dimension. Cela a l'avantage de recaler les nuages de points apparus sur une zone vue par plusieurs capteurs et de corriger l'effet zoom apparaissant obligatoirement du fait de la position variable de l'objet par rapport à l'ensemble des capteurs.

Le calcul de la troisième dimension étant basé sur la recherche de points similaires, facilité par le positionnement prédéfini des capteurs mais particulièrement difficile pour des surfaces uniformes comme le sont les surfaces dentaires, est associé à ces trois capteurs des LEDs dans le blanc pour révéler la couleur réelle de l'objet mesuré. Les points images renferment donc deux informations complémentaires, le temps (moment de la prise de vue qui deviendra commun à l'ensemble des deux vues pour simplifier les calculs), et sans que cela soit obligatoire, une cinquième dimension, elle même divisible dans un système chromatique, et qui correspondra à l'information couleur.

Avantageusement ces LEDs peuvent aussi être d'une longueur d'onde (de quelques nanomètres) prédéfinie permettant de mettre en évidence des marquages naturels (fonds de sillons ou zones de couleurs différenciant tumeurs, gencives ou teintes des dents) ou des marquages réalisés avant la prise d'empreinte, faits à l'aide de marqueurs de couleurs complémentaires.

Avantageusement ces marquages peuvent être des objets de différentes formes placés (dans la zone mesurée), collés ou logés (dans les espaces inter dentaires ou sur les têtes d'implants) sur l'objet analysé.

Avantageusement, cette ou ces LEDs sont placées autour de chaque lentille de focalisation de l'image placée devant le capteur.

Avantageusement ces LEDs peuvent être une association entre les LEDs blanches et de longueur d'onde prédéfinie.

Selon un troisième mode de réalisation, les capteurs sont répartis sur toute ou partie d'une arcade dentaire afin de réaliser une empreinte en un seul cliché, évitant le balayage clinique de l'arcade du patient par l'opérateur. Ils sont ainsi disposés dans une sorte de porte-empreinte optique permettant la saisie complète de l'arcade en un seul cliché. Cette configuration étant permise par la simplicité du dispositif selon l'invention.

Les LEDs sont reparties le long du porte-empreinte et sont commandées ensemble ou spécifiquement suivant l'analyse en cours et tel que cela est défini dans le premier mode de réalisation.

La bouche présentant des ouvertures variables, il est possible que l'encombrement de la tête d'analyse du montage précité ne permette pas son introduction jusque dans les zones inter dentaires profondes (zones molaires). Aussi et avantageusement, la présente invention propose, selon un quatrième mode de réalisation, une variante composée d'endoscopes uniquement conduits d'image, à la différence des autres systèmes dentaires, dont la lentille de tête se trouve dans la même position que les capteurs préalablement décrits. Dans ce cas l'image est :

- soit reflétée par un miroir ou un prisme qui renvoie l'image de la zone mesurée sur le capteur, lui-même pouvant comporter un système de lentille de telle sorte que le plan focal image se trouve dans le plan occupé par le capteur.

- soit conduite par une fibre optique "image" réduisant et simplifiant le montage optique de l'endoscope.

Dans ces deux cas, les LEDs peuvent se trouver dans le corps de la caméra et la lumière est alors conduite par une fibre optique, ou alors elles sont reportées autour de la lentille frontale, à l'extrémité de l'endoscope.

Avantageusement ces endoscopes peuvent être d'un nombre supérieur au nombre de capteurs. Dans ce cas un même capteur peut se trouver en face de plusieurs endoscopes visualisant différentes zones du corps humain analysé. Cela a l'avantage de réduire la partie électronique de la caméra et/ou d'augmenter la quantité des points de vue image lors de la prise de vue.

Pour préserver la qualité de chacune des images il est possible:

- soit décaler le temps, par exemple de quelques 1000e de secondes, de la lecture de l'image de chaque endoscope à l'aide d'un obturateur mécanique ou électronique..

- soit de travailler à des fréquences, modulations ou intensités d'image différentes évitant un décalage entre chacune des vues propres à chacun des endoscopes intéressant un seul capteur.

Enfin, et sans que cela soit limitatif, il existe une variante particulière à tous les modes de réalisation, qui utilise un micro miroir type "micro miror DMD Discovery" de Texas Instrument ®, qui associé à la carte ODLP permet de diriger les images selon un angle prédéfini. Cette technologie, utilisée largement dans les projections des vidéos projecteurs miniaturisés, sert, dans la présente invention, de capteurs d'images à différents angles.
Ainsi, selon un sixième mode de réalisation, le dispositif selon l'invention, comporte un tel micro miroir associé plusieurs capteurs CCD ou Cmos et aux LEDs.
Ce micro miroir permet de prendre x prises de vues, sous des angles différents, et à des vitesses extrêmement élevées, sans bouger la caméra. Le micro miroir, placé derrière l'optique de focalisation renvoie l'image sur tout ou partie du capteur CCD ou Cmos, en suivant un chemin optique d'images simple et direct. La première image est donc réfléchie suivant un angle prédéfini. Immédiatement après le micro miroir change d'angle et reflète une nouvelle image légèrement décalée et sous un angle différent de la première image sur le même CCD ou Cmos, après rafraîchissement, ou sous une autre partie du même CCD ou encore sur un nouveau CCD ou Cmos, positionné en fonction des angles prédéfinis par la position angulaire du micro miroir.
Il est possible de répéter ces opérations un grand nombre de fois en faisant simplement varier l'angulation du micro miroir. Ce montage a l'avantage de permettre l'utilisation d'un seul système optique à la tête de la caméra, un seul miroir offrant différents angles de réflexion d'images, et éventuellement un seul capteur, se trouvant ou non devant un système de conduction d'images pouvant être une fibre ou un système de lentilles.
Il va de soi que plus les images sont nombreuses sous des angles différents, et plus la mesure est précise. C'est pour cette raison que la présente invention ne se limite pas à l'utilisation d'un seul micro miroir mais éventuellement à plusieurs, ce qui a l'avantage de multiplier le nombre de vues. Ainsi, si le système renferme trois capteurs, comme défini dans le premier mode de réalisation, et si quatre angles de visualisation des micro miroirs sont choisis (90°, 92°, 94° et 96°) on obtient en une seule vue douze images différentes à des angles de vue différents en quelques millièmes de secondes.
Ainsi la présente invention peut être définie comme étant un dispositif de prise de vue dentaire en trois dimensions sans utiliser la projection de lumière structurée, à la différence de tous les systèmes existants, et composé d'un ou plusieurs systèmes optiques de focalisation de une ou plusieurs vues, associé directement à plusieurs capteurs CCD ou Cmos, ou éventuellement indirectement afin de limiter l'encombrement de la tète de la caméra, par l'intermédiaire d'un prisme, miroir ou des micro miroirs conduits d'images endoscopiques par des lentilles ou des fibres, sous un éclairage à LED blanche ou/et de valeurs chromatiques définies.
Reste bien entendu l'approche mathématique de ce qu'on appelle le petit b/h, c'est à dire la mesure de la troisième dimension z, dans la mesure où x et y sont définis par leur position sur les pixels des capteurs, lorsque l'espace entre les deux centres optiques des lentilles de focalisation sont faibles par rapport à la distance h de la profondeur de champ au centre optique de ces lentilles.
le modèle utilisé pour un faible b/h est:

$$I\ right\ (x) = \lambda\ (x)\ .\ I\ left\ (x + \epsilon\ (x))$$

où $\epsilon$ est la déformation géométrique.

$$\partial z = \partial\epsilon / b/h$$

sur ces bases est utilisé le principe de Shannon pour résoudre les problèmes de disparité des mesures dues à la sub pixelisation.

$$\int u\ (x + \mu m)\ .\ u(x)\ .dx$$

$$\rho x_0 \;(\mu m) = \frac{\varphi \cdot x0}{\|u(. + \mu m)\|\,\varphi_{x0} \cdot \|\breve{u}\|\,\varphi_{x0}}$$

où

$$\breve{u} = \int \rho x_0(x) \cdot u^2(x) \cdot dx$$

Les avantages et les caractéristiques du dispositif selon l'invention, ressortiront plus clairement de la description qui suit et qui se rapporte au dessin annexé, lequel en représente plusieurs modes de réalisation non limitatifs.

Dans le dessin annexé :

- la figure 1 est une vue schématique en perspective et en éclaté du dispositif de mesures tridimensionnelles et temporelles selon l'invention.
- les figures 2a, 2b, 2c, 2d et 2e, représentent des vues schématiques en une coupe longitudinale d'une partie du même dispositif, selon des modes de réalisation différents.
- la figure 2f représente une vue schématique en perspective d'une partie de la figure 2e.
- les figures 3a, 3b, 3c, 3d et 3e représentent des vues en coupe transversale de la même partie, selon des modes de réalisation différents.
- les figures 4a, 4b et 4c représentent des vues de différentes configurations du dispositif selon l'invention utilisé en dentisterie.
- les figures 5a, 5b et 5c représentent des vues schématiques de détails des figures 4a, 4b et 4c.
- la figure 6 représente une illustration schématisée des différentes étapes du traitement de la mesure dentaire utilisant le dispositif, objet de l'invention.

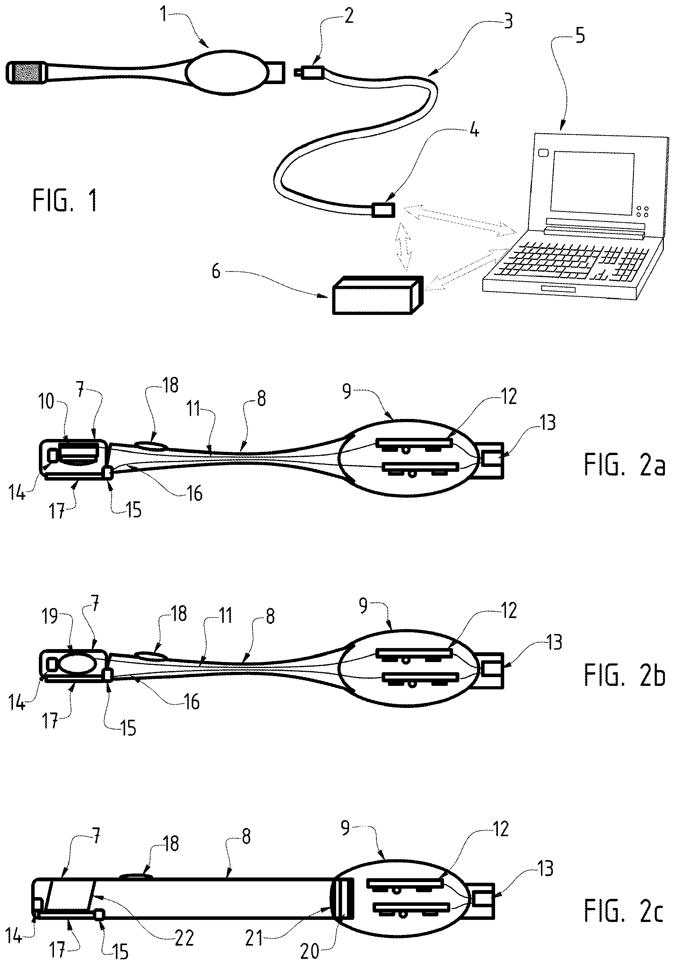

Comme visible sur la figure 1, le dispositif selon l'invention comprend une caméra à focale fixe 1 utilisant la technologie décrite dans l'invention, une connexion 2 entre la caméra 1 et un câble 3 servant d'alimentation et de transfert des données, une connexion 4 entre le câble 3 et un ordinateur 5 étant de type USB et un boîtier 6, pouvant être placé en intermédiaire servant à l'adjonction de carte de pilotage du processeur de la caméra et /ou de traitement de l'image si celles ci ne sont pas placées dans la caméra ou dans l'ordinateur.

Cette même caméra 1 peut utiliser une connexion de transmission d'images, ou de données issues des images, sans fil type Wi fi et un système de recharge pour batteries rechargeables pour l'énergie à apporter à la caméra.

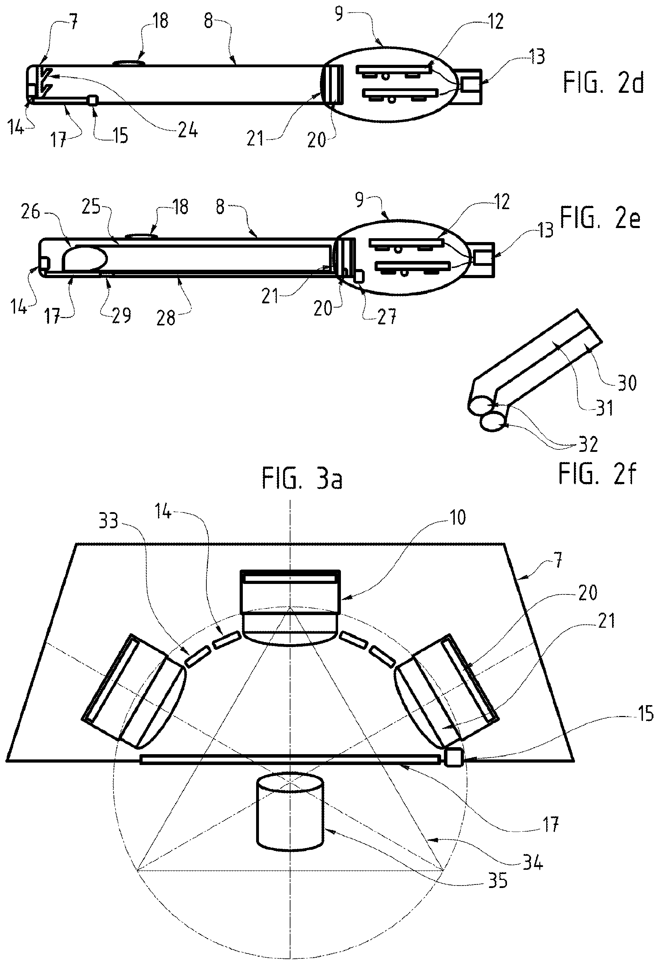

Pour détailler chacune des parties de cette invention, nous nous reporterons aux figures 2a, 2b, 2c, 2d et 2e qui présentent une option clinique dentaire sous son aspect fonctionnel.

Le dispositif, présenté sur ces figures, comporte une caméra de mesure couleur tridimensionnelle, comportant la saisie totale d'une ou plusieurs vues de l'objet mesuré, avec un système de focalisation fixe, stable, pré calibré et immobile, sans aucune correction dynamique de focalisation ou sans projection de lumières structurées projetées ou balayant l'objet mesuré, type points, lignes, grilles à pas connus fixes ou déplacées, disposant de LEDs d'éclairage par lumière blanche ou à longueurs d'onde connues et définies préalablement permettant de mettre en évidence les couleurs que l'on désire extraire de l'image mesurée. Les capteurs, eux- mêmes en position fixe, stable, pré calibrée et immobile sont placés derrière le système optique, ici au nombre de deux afin d'observer l'ensemble de l'objet mesuré selon un angle différent préalablement défini au moment de la calibration de la caméra durant sa fabrication. A la différence de tous les systèmes existants, la présente caméra 1 ne comporte aucune division de l'image saisie, aucun mouvement mécanique de focalisation et aucune projection de lumière structurée. C'est l'association judicieuse des lumières délocalisées, c'est-à-dire ne venant qu'éclairer la scène, qui permet de mettre en évidence les éléments intéressants pour l'observateur et les analyses qu'il souhaite faire en terme de mesure ou de diagnostic.

Ces caractéristiques sont réputées fixes et non modifiables par l'opérateur à l'exception du type d'éclairage sélectionné, encore que cette fonction peut être pilotée par une succession d'actions automatiques conduisant au diagnostic désiré. Pour ce faire, l'opérateur (dentiste ou prothésiste) dispose d'un ordinateur lui indiquant les opérations que peut exécuter la caméra et lui permettant de faire le choix entre une fonction et une autre.

Ainsi en fonction "mesure", après avoir sélectionné ce mode d'action, l'opérateur lancera la mesure, à l'aide d'un bouton situé sur la caméra, ou d'une pédale reliée à l'ordinateur ou au boîtier intermédiaire, après avoir positionné la caméra sur la zone à mesurer et l'arrêtera lorsqu'il jugera avoir assez d'informations il cessera la pression ou appuiera une deuxième fois. Le fait d'avoir une image colorée permet à l'opérateur de disposer d'analyse automatique des zones dentaires (blanches en général) et gingivales (rouges en général) ce qui est impossible dans les méthodes actuelles utilisant les projections par lumières structurées. De même par le positionnement d'index de couleurs connues il a la possibilité de faire des analyses discriminatives pour la reconnaissance d'objets dans l'image mais aussi leur position (têtes d'implants ou de tenons, brackets d'orthodontie...) ou encore pour faciliter la corrélation des vues (repères, tracés colorés sur l'objet ou couleurs sélectives comme les fonds de sillons ...)

Ainsi dans la fonction diagnostic il sélectionnera sur l'ordinateur le type de diagnostic désiré, par exemple mélanome, et la caméra lancera un balayage de longueur d'onde correspondant à la mise en évidence des zones intéressant les longueurs d'onde présélectionnées et présentes sur une image 3D. En plus de cela, et grâce à l'analyse 3D de l'objet, le recouvrement des mesures dans le temps permettra de mieux suivre l'évolution de la dite pathologie. Il est en effet admis par les professionnels que l'étude d'une image suspecte peut être faite en 2D mais c'est surtout l'évolution de son volume et de sa couleur qui sert de référence au suivi dans le temps de sa dangerosité. Le fait de disposer d'un volume référé à un centre mathématique (comme par exemple le barye centre) permet de superposer les images sur un centre dépendant de l'objet et non pas de l'observateur afin d'en apprécier objectivement l'évolution du volume, l'analyse de la couleur venant se reporter sur une forme 3D, ce qui n'est pas le cas aujourd'hui des méthodes pratiquées sur des surfaces 2D ou celles utilisant des lumières ou les ondes structurées (OCT, scanner ou IRM).

Ainsi dans la fonction analyse de couleur, après avoir sélectionné cette fonction, l'analyse de la couleur du volume mesuré et reporté sur celui-ci pourra se faire sur des bases ne dépendant pas du méta mérisme dépendant de l'éclairage présent dans la pièce de l'opérateur. Le fait de disposer de plusieurs longueurs d'onde permettra de se rapprocher d'un spectre continu et de disposer d'une analyse spectro colorimétrique. Pour simplifier cette opération, il sera possible de ne se reporter qu'aux trois couleurs RGB complémentaires et effectuer une simple analyse colorimétrique.

Enfin, et ceci n'est pas limitatif, le fait de disposer de deux images 2D pour construire l'image 3D permet, en temps réel, de basculer la vision sans modification de la caméra à des visualisations 2D couleurs comme l'ensemble des caméras disponibles sur le marché de la dentisterie aujourd'hui. Cette caméra permet donc, du fait qu'elle n'utilise pas de projection de lumières structurées, d'effectuer toutes les fonctions connues aujourd'hui incluant les effets zoom, mais aussi les applications de diagnostics couleurs sur des images 2D comme les détections de caries par fluorescence dans les verts, bleus ou ultra violet (de 500 à 300 nm) ou les visualisations dans les rayonnements rouges et infrarouges (de 600 à 900 nm), suivant les LEDs qui auront été émulées dans l'analyse.

Ce même effet zoom en image couleur ou les émulations pourront être faites sur les images 3D. Il va de soi que le passage de la couleur au niveau de gris ne sera qu'une fonction offset présente dans les logiciels pilotant les traitements d'images issus du fonctionnement de la caméra.

La connexion entre la caméra et l'ordinateur peut se faire avec ou sans fil.

Selon l'invention la connexion par fil 3 sera préférentiellement par l'intermédiaire d'une connexion USB 4 autoalimentée avec un port spécifique 2 côté caméra 1. Cette connexion spécifique 2 sera conçue de telle manière qu'elle sera adaptable à toutes formes de caméras qui, comme cela sera vu plus loin peuvent revêtir différents aspects.

De même manière, et selon l'invention, la connexion pourra être sans fil, en mode, par exemple et ceci n'est pas limitatif, par Wifi. Dans ce cas l'antenne sera incluse dans la caméra ou connectée à la place de la connexion spécifique 2. De même, sur l'ordinateur sera introduite dans la connexion USB une antenne d'envoi et de réception des données correspondant aux ordres donnés par le programme situé dans la caméra ou dans l'ordinateur. Cette disposition permettra une communication rapide, conviviale et aisée quelque soient les configurations des cabinets médicaux, dentaires ou des laboratoires de prothèse.

Selon l'invention, l'ordinateur 5 est de type standard avec un écran, inclus ou détaché. Cet ordinateur utilise des cartes standard spécialement programmées pour piloter la caméra ou des cartes spécifiques de pilotage qui sont placées sur le bus.

Dans le cas où l'ordinateur ne pourrait pas être équipé ou s'il est préalablement présent dans l'unité de soin, un boîtier intermédiaire 6 est positionné entre la caméra et l'ordinateur pour suppléer à ce manque. De la même manière et pour la même fonction, ce boîtier sera positionné en aval de l'ordinateur, et la connexion USB 4 de la connexion sera directement branchée sur le port USB de l'ordinateur sans intermédiaire.

Dans la configuration représentée sur la figure 2a, une section présente la tête 7 de la caméra 1, un bras 8 permettant son introduction en bouche et un corps 9, souvent à l'extérieur de la bouche. La tête présente la section de l'ensemble optique d'une lentille centrale 10 du système optique comprenant trois ensembles (lentilles de focalisation fixes, c'est-à-dire sans réglage de focalisation avant la prise de vue, et capteurs CCD ou Cmos) connectés à la carte de connexion d'images 12 par un câble 11 de préférence blindé pour éviter toutes interférences néfastes à la qualité de l'information transmise. Cette carte est elle-même reliée à l'ordinateur 5 ou au boîtier spécifique 6 grâce au connecteur spécifique 13 dépendant de la caméra 1. Cette même section longitudinale permet de distinguer les LEDs placées vers le système optique 14 à l'intérieur de la tête protégée par la glace de protection et/ou en périphérie du système optique, en dehors de celui-ci 15. Un bouton 18 permet d'actionner la prise de vue, si on n'utilise pas la pédale de commande. Le fait d'utiliser un système de prises de vues sans décalage permet de prendre cette image 3D avec le bouton sans risquer le flou pouvant être créé par un mouvement involontaire.

La conception de cette caméra a pour objectif de recouvrir une champs de lecture relativement étroit (un volume de 15x15x15 cm) avec une précision de quelques µm. Pour cela le champ balayé par les systèmes de vision sont identiques mais les points de vues ont un angle légèrement différents.

Dans la configuration représentée à la figure 2b, on voit une coupe longitudinale avec seulement deux systèmes optiques dont on ne voit ici que la section d'un des deux systèmes 19 et les LEDs 14 et 15 pouvant occuper les mêmes positions que décrites précédemment. Cette configuration permet de réduire significativement le volume de la tête de la caméra mais oblige à un développement software plus conséquent.

Dans la configuration représentée à la figure 2c les capteurs CCD ou Cmos 20, et les systèmes de focalisation fixes 21 placés cette fois-ci dans le corps de la caméra, sont positionnés en face de miroirs réflecteurs d'images. Ces miroirs, qui peuvent être au nombre de deux ou plus, dont on voit une partie de l'un d'eux 22 ont une angulation telle qu'ils peuvent couvrir la même zone de lecture sous des angles légèrement différents permettant au capteur d'enregistrer la même scène avec un léger décalage spatial.

Dans la configuration représentée à la figure 2d les capteurs CCD ou Cmos 20, et les systèmes de focalisation fixes 21 sont toujours placés dans le corps de la caméra, mais les miroirs sont positionnés en face de micro miroirs 24 pilotés par la carte processeur, par exemple de type FPGA situés dans la caméra 1 dans l'ordinateur 5 ou dans le boîtier intermédiaire 6. On sait que les micro-miroirs, très utilisés pour la projection d'images dans les projecteurs vidéos, le sont rarement dans les techniques de visualisation. Ces micro miroirs pouvant être orientés dans les trois directions de l'espace et ce, très rapidement, un seul miroir peut remplacer une dizaine de miroirs, et même plus, permettant de voir une scène légèrement différente, dont une partie commune le sera sous des angles différents. Les DMMD (ou digital Micro Mirror Device) appelés aussi DLP (Digital Light processing), par exemple de Texas Intrument, utilisent des micro miroirs ( par exemple 10µmx10 µm) dont l'angle change rapidement sous l'effet de variation du voltage ou de la tension. Leur nombre étant supérieur à 1024 x 1280, la précision annoncée est possible. Le voltage de modification d'angle est envoyé à l'adresse des électrodes indiquant la torsion à appliquer à la rotule supportant et orientant le miroir. Une inversion de polarité est suffisante pour mettre le miroir dans une autre position. Les miroirs sont tellement petits que le changement se fait en moins de 15µs.

Cette solution est tout particulièrement intéressante car elle permet de sectoriser les vues et de les enregistrer facilitant ainsi les corrélations des images durant la phase de traitement d'images.

Il va de soi que cette même configuration peut se concevoir, selon l'invention, avec le capteur situé dans la tête ou dans le bras de la caméra, évitant ainsi une déperdition de l'image ou un bras trop important risquant de gêner les insertions dans des zones étroites. De même un seul capteur peut être sectorisé en surface afin qu'il soit en mesure de recevoir plusieurs vues en même temps.

Enfin dans la configuration représentée à la figure 2e, des capteurs CCD ou Cmos 20 et leur système optique 21 placés dans le corps de la caméra, sont positionnés en face de fibres optiques de transmission d'images 26 dont l'orientation de la section de sortie ou la torsion appliquée à l'extrémité, visible sur la figure 2f, 30 et 31, permet de recouvrir un champ identique sous des angles différents 32. Ces fibres peuvent être en nombre multiple permettant ainsi un captage sous plusieurs angles, et les systèmes optiques et capteurs peuvent être positionnés dans la tête ou dans le manche pour éviter les réductions du signal image.

On notera que, pour toutes les configurations représentées sur ces figures, les LEDs peuvent être positionnées dans la tête de la caméra 14 ou dans le corps de celle ci 27. Dans ce dernier cas elles sont placées en face d'une fibre transmetteuse de lumière 28 ayant son extrémité, projetant la lumière, située à la tête de la caméra 29.

En référence maintenant à la figure 3a, on peut voir une représentation du dispositif selon l'invention utilisant, dans la tête de la caméra 7, trois systèmes optiques fixes 10 et équilatéraux 34 de focalisation de l'image 21 positionnés autour de l'objet mesuré 35 et placés à une distance fixe et prédéterminée par calibration devant les capteurs CCD ou Cmos 20 placés à l'extrémité du boîtier, sans recours à des endoscopes ou de miroirs de réflexion et selon la configuration à trois capteurs laissant voir la position possible des LEDs d'éclairage spécifique 14 ou de lumière de longueurs d'onde spécifiques 33. Comme on le voit sur cette section, ces mêmes LEDs 15 peuvent aussi être placées autour de la fenêtre 17 ce qui permet de mieux éclairer la scène mesurée.

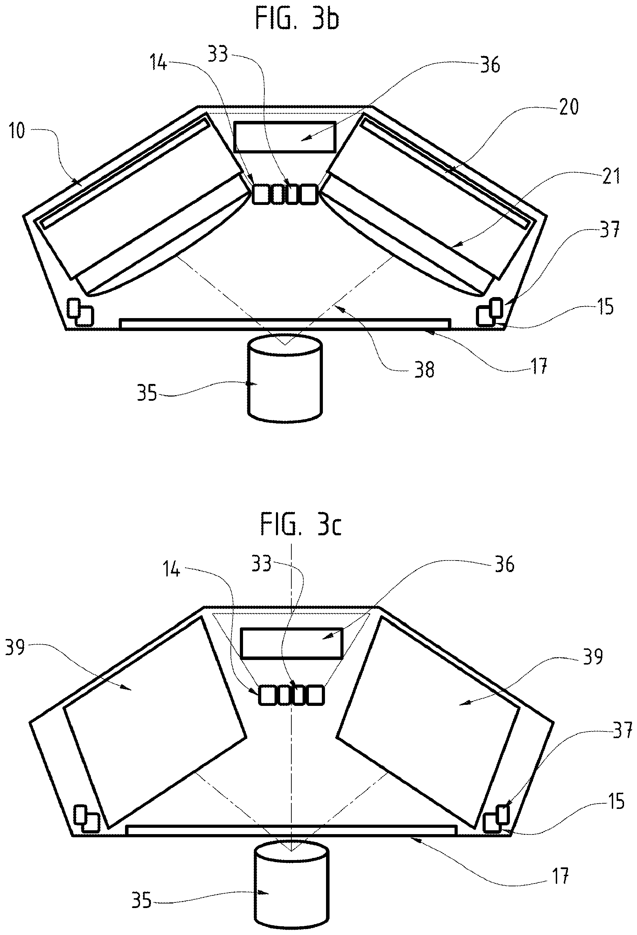

En référence maintenant à la figure 3b, on peut voir une coupe frontale de la configuration de la figure 2b, les deux systèmes optiques fixes de focalisation de l'image 21 sont positionnés autour de l'objet mesuré et placés à une distance fixe et prédéterminée par calibration devant les capteurs CCD ou Cmos 20 placés à l'extrémité du boîtier, sans recours d'endoscopes ou de miroirs de réflexion. Nous retrouvons les LEDs elles- même en position centrale temps pour les LEDs de lumière blanche 14 que pour les LEDs à longueur d'onde spécifiques 33. Ces LEDs 15 et 37 peuvent aussi être placées autour de la glace protégeant l'ensemble optique 17. La position ici est sous la forme particulière d'un triangle isocèle 38 mais ceci n'est pas limitatif. Toutes positions des systèmes optiques 10 sont envisageables dans la mesure où la configuration est figée dans le logiciel de mesure et de corrélation des vues. Dans cette même figure, du fait de la réduction du volume des éléments optiques, peut être placé un système de traitement d'images 36 (image processing), à proximité des capteurs ce qui évite les déperditions de l'information le long des fils de connexion.

En référence maintenant à la figure 3c, on peut voir que des miroirs de réflexion de l'image 39 peuvent être au nombre de deux ou plus sont positionnés dans la tête de la caméra selon des angles différents et permettent le captage de l'image sur un ou plusieurs capteurs CCD ou Cmos 20 placés derrière les systèmes de focalisation 21 dans le corps de la caméra 1, eux - même positionnés en face des miroirs conductions d'image concernés. Nous retrouvons les différentes LEDs 14-17-33 et 37 ainsi qu'éventuellement le système de traitement de l'image 36.

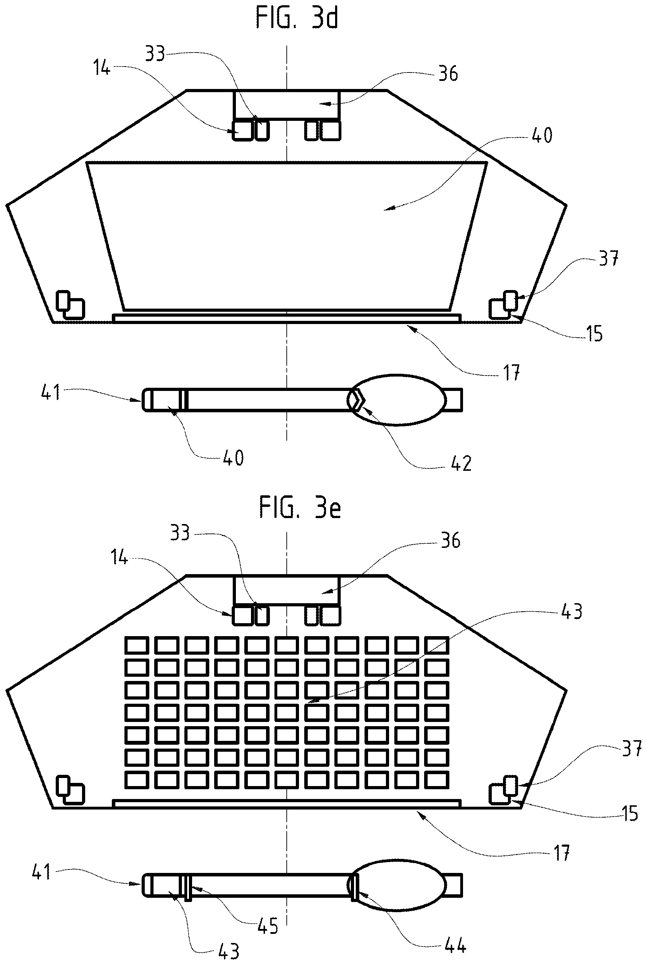

Une variante est proposée sur la figure 3d. Il s'agit dans ce cas de n'utiliser qu'un seul miroir 40 d'anguler légèrement les systèmes optiques 10 incluant les systèmes de focalisations 21 et les capteurs 20. On retrouve cette disposition très particulière dans le dessin complémentaire où l'on voit au travers de la vitre de protection l'unique miroir 40 situé dans la tête de caméra et la position des deux capteurs 42 faisant un angle relativement faible, de quelques degrés, de telle sorte que la surface du miroir soit nécessaire et suffisante pour refléter une image les deux angles différents correspondant à la position des deux capteurs.

Il va de soi, et selon l'invention, que ce montage est un cas particulier et qu'il peut être composé de plus de 1 miroir et de plus de deux capteurs.

En référence maintenant à la figure 3e, correspondant à la configuration de la figure 2d, nous voyons les micro miroirs 43 disposés à la place du miroir statique unique 40. Nous pourrions utiliser plusieurs composants DMMD mais un seul est suffisant. La multiplication permet de multiplier les images et d'augmenter la précision de la mesure tout en simplifiant les algorithmes de recherche de la troisième dimension.

Dans ce montage, un seul ensemble système de focalisation 21 et CCD ou Cmos 20, sans que ce soit limitatif, se trouve dans le corps de la caméra 44 ou en position directement proche des micro miroirs, dans la tête de la caméra 45.

Nous pouvons aussi, dans les montages décrits dans les figures 3c et 3e conduire l'image à l'aide de fibres optiques transmetteuses d'images tel que montré dans la configuration de la figure 2e. Dans ce cas une ou plusieurs fibres optiques sont placées devant les miroirs, les micro miroirs ou directement en direction de l'objet mesuré (figure 2f), éventuellement derrière un système optique et dans l' axe du ou des capteurs CCD ou Cmos placés dans le corps de la caméra, eux -même positionnés en face de l'autre extrémité des fibres optiques de transmission d'image. Il est possible aussi d'utiliser des fibres très courtes et de placer les systèmes optique + capteur 10 dans la tête de la caméra, ce qui limite significativement le montage et le réglage du système.

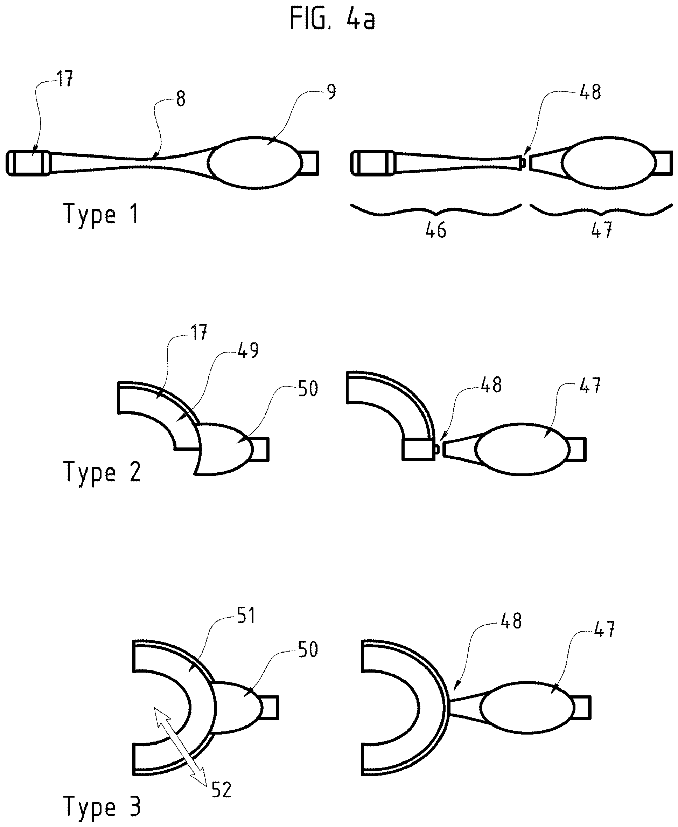

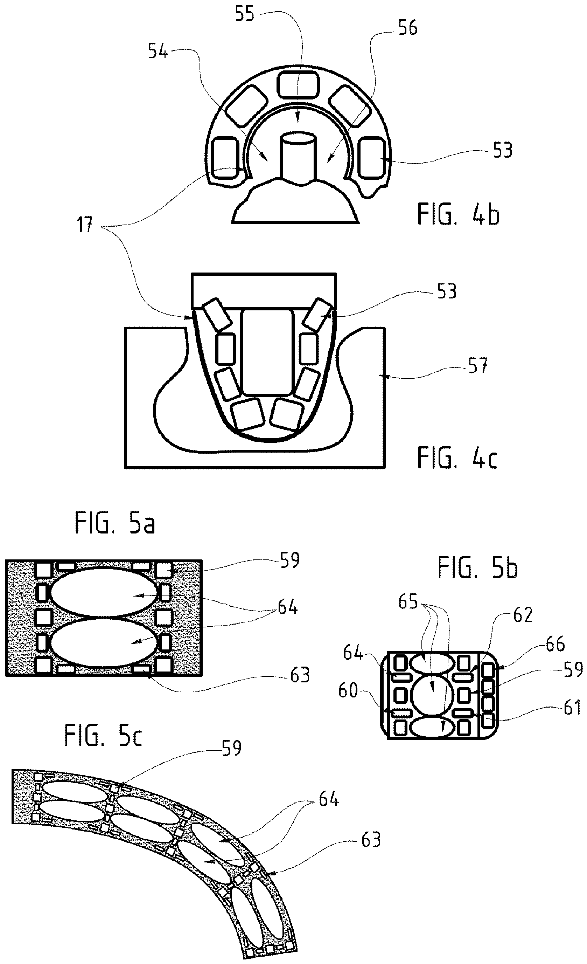

Les figures 4a, 4b et 4c démontrent combien la présente invention répond à des besoins cliniques jusqu'alors restés sans réponse en dentisterie et ophtalmologie.

En dentisterie, le praticien qui souhaite faire un diagnostic ou une empreinte dans le but de réaliser une prothèse ou un implant a besoin de deux types d'approche, l'une rapide lui donnant seulement les informations nécessaires (en terme de surface mesurée et de précision apportée) et l'autre complète et précise. Par exemple la réalisation d'une couronne sur une molaire mandibulaire est réalisable par CFAO dentaire si l'empreinte optique de la zone de préparation est précise, complète et soignée si l'empreinte optique des dents antagonistes donne au moins les mesures des points de contacts (cuspides, sillons) et des formes d'arcades ce qui ne nécessite pas la même attention. De même une empreinte pour un appareil de redressement des dents (orthodonties) ne nécessitera pas autant de précision que celle se rapportant à la réalisation d'un bridge céramique sur des têtes d'implants.

C'est la raison pour laquelle le dispositif selon l'invention est décliné en trois types de caméras dentaires.

Le type 1, schématisé dans la figure 4a représente le dispositif de l'invention en dentisterie permettant la prise de plusieurs vues mais obligeant au déplacement de la tête de mesure. Il s'agit d'une caméra qui filme en 3D et en couleur les arcades dentaires en utilisant le principe de l'invention à savoir sans modification ou ajustage de la focale et sans utilisation de lumière structurée mais en utilisant la projection de lumières LEDs afin de favoriser la reconnaissance des tissus et des pathologies. Elle peut être d'une seule pièce, ou se diviser en deux parties, l'une appelée "tête de mesure" 46 comportant le système optique (lentilles + capteurs CCD ou Cmos) associé aux LEDs, et une deuxième partie 47 appelé "corps de traitement" contenant les cartes de contrôle et de traitement d'images disponibles pour, éventuellement, d'autres formes de "tête d'empreinte optique" connectables sur une prise spécifique 48 réunissant sur un corps universel 47 plusieurs types de "tête d'empreinte" comme la tête de caméra 46.

Le type 2 est composé du même montage d'unités de base, présent dans la tête de la caméra mais multiplié et réparti dans une sorte de porte- empreinte optique 49 pour une hémi arcade. Ce montage est rendu possible par le fait que n'ayant qu'un seul conduit image, sans conduit pour la lumière structurée, nous pouvons multiplier le long de l'hémi arcade, derrière la glace de protection 17 le montage se trouvant à l'extrémité de la caméra et de ce fait ne pas être obligé de parcourir l'arcade pour avoir l'information. Un seul cliché permet de saisir toute la surface dentaire se trouvant dans l'axe de prise de vue, ne comprenant pas les contres- dépouilles. Cette prise de vue est rapide et suffisante pour avoir une mesure ou une information complète sans provoquer de gène pour le patient.

La partie traitement d'images peut être incorporée au porte- empreinte 50, ou celui-ci peut être déconnectable suivant une prise 48 analogue à cele du type 1, ce qui permet à ce porte -empreinte d'être peu coûteux, plus simple et d'utiliser le même "corps de traitement" contenant les cartes de contrôle et de traitement d'images 47 que le type 1.

La connexion peut se faire par l'intermédiaire d'un câble complémentaire ou un système sans fil par exemple Wifi, réunissant le connecteur 48 et le boîtier caméra 47, non présenté sur le dessin, afin de faciliter la manipulation du petit système de lecture et prise d'empreinte.

Le type 3 est une extension du type 2 pour l'ensemble de l'arcade, ayant une vitre de protection de la forme de l'arcade complète 51, protégeant les systèmes optiques pré positionnés comme dans le système des figures 2a et 2b.

Comme le type 2, il est réalisé en une seule pièce avec un système de traitement d'images situé dans le manche 50 ou apparait déconnectable grâce à une prise spécifique 48 lui permettant de bénéficier du système de traitement d'images interchangeable 47.

Ce type 3 sera très utile pour les grandes reconstitutions occlusales correspondant à une prothèse sur l'arcade opposée ou pour certains diagnostics ne nécessitant pas une vue des zones en contre- dépouille. Il trouvera aussi une application majeure en étant utilisé avantageusement pour la corrélation des vues radiologiques ou cône beam et ainsi faciliter le rassemblement des informations issues de l'empreinte optique et de l'empreinte radiologique. Technique et demande très importante dans le domaine de l'implantologie, ce type 3 simplifiera considérablement la procédure et assurera le lien tant attendu entre la radio clinique et l'exercice du prothésiste en CFAO dans son laboratoire.

Nous savons en effet que dans les empreintes radiologiques en 2D type Scanner ou Cone bean, ou 3D type IRM, l'information de la surface extérieure de la gencive et des dents existe mais n'est pas précise. La connaissance de cette même surface à l'aide de l'empreinte optique issue de notre invention, permet de réunir les deux fichiers afin de disposer d'un ensemble complet, cohérent et précis permettant la réalisation des implants en toute sécurité, et de disposer des logiciels de CFAO dentaires existants.

Une section 52 de la figure 4a, type 2 ou 3, permet de voir deux variantes de la disposition décrite dans les figures 3a et 3b. Il est possible, de plus en plus, de réduire les volumes des systèmes optiques associant capteurs et lentilles de focalisation, comme le montre les caméras Web incluses dans les écrans des ordinateurs portables. Comme notre système de focalisation est figé et précis par rapport à un volume et une profondeur de champ définie, nous proposons donc, selon l'invention, de miniaturiser et de multiplier les capteurs afin de bénéficier d'une analyse de toute la surface étudiée, y compris les contres-dépouilles en distribuant ces ensembles le long de l'empreinte optique à réaliser.

La figure 4b représente donc une variante de ces systèmes type 1, 2 et 3 avec des têtes de lecture (lentilles de focalisation et capteurs) miniaturisées 53 entourées de LEDs non visibles dans le dessin et protégées par une vitre 17. Cette variante enveloppe toute ou partie de l'arcade, et permet de prendre des vues des surfaces vestibulaires 54, occlusales 55 et linguales 56 en un seul cliché. Il s'agit donc d'un type de caméra spéciale plus enveloppante, plus volumineuse mais capable de prendre une vue complète et précise des zones avec et sans contre-dépouille. Elle est utilisable suivant la configuration type 1 localisée à une zone d'arcade, type 2 pour une hémi arcade ou type 3 pour toute l'arcade. La prise de vue est complète, totale et très rapide. Les corrélations et les mesures sont facilitées par la connaissance a priori par les algorithmes de traitement d'images de la disposition et la fixité des systèmes optiques et des systèmes de focalisation, objet de l'invention. Ce type 4b est un montage utilisable en bouche mais c'est surtout dans son application sur les modèles de travail en plâtre qu'il trouvera toute son application, dans les cabinets dentaires et dans les laboratoires. La prise d'empreinte est dite empreinte optique centrifuge car elle converge vers le centre de l'image.

La figure 4c montre une autre variante en forme convexe pour la prise d'empreinte dans les porte-empreintes traditionnels 57, évitant ainsi au dentiste d'effectuer la coulée de son modèle mais lui permettant de faire une empreinte classique, s'il le préfère et de la transmettre au laboratoire sous une forme numérique (support solide ou internet). La prise d'empreinte est dite empreinte optique centripète car elle converge vers l'extérieur de l'image.

Les figures 5a, 5b et 5c représentent des vues détaillées de la fenêtre optique du système de visualisation en dentisterie pour une caméra avec deux systèmes optiques 64 sur la figure 5a, avec trois systèmes optiques 65 sur la figure 5b, et pour une hémi arcade sur la figure 5c. Dans ces figures est visualisé un mode de positionnement, mais ceci n'est qu'un exemple, en les détaillant nous pouvons voir la position des conduits images 58, qui peuvent être des lentilles ou des miroirs et la position possible des LEDs "blanches" 59 et des LEDs de longueurs d'onde spécifiques, comme, par exemple et ceci n'est pas limitatif, des LEDs dans le rouge et/ou infrarouge 60, dans l'orange 61 dans le vert 62 dans le bleu et/ou ultraviolet 63 .

Comme nous le voyons dans la figure 5b, les LEDs blanches 66 peuvent être positionnées à la périphérie de la fenêtre de protection 17.

Chacune de ces LEDs a un rôle spécifique que nous décrirons plus complètement dans la suite du texte. Les LEDs dite "blanches ou lumière du jour" ont pour objet de relever les couleurs "vraies" perçues par l'oeil humain, avec exactitude en favorisant le rapport signal/bruit alors que les LEDs à des valeurs de longueurs d'onde prédéterminées ont pour fonction de mettre en évidence des zones intéressantes sur les plans mathématique (zones de corrélation...), pathologique (réactions de pathologie, de fluorescence ...) ou anatomique (la gencive est rouge et les dents sont blanches).

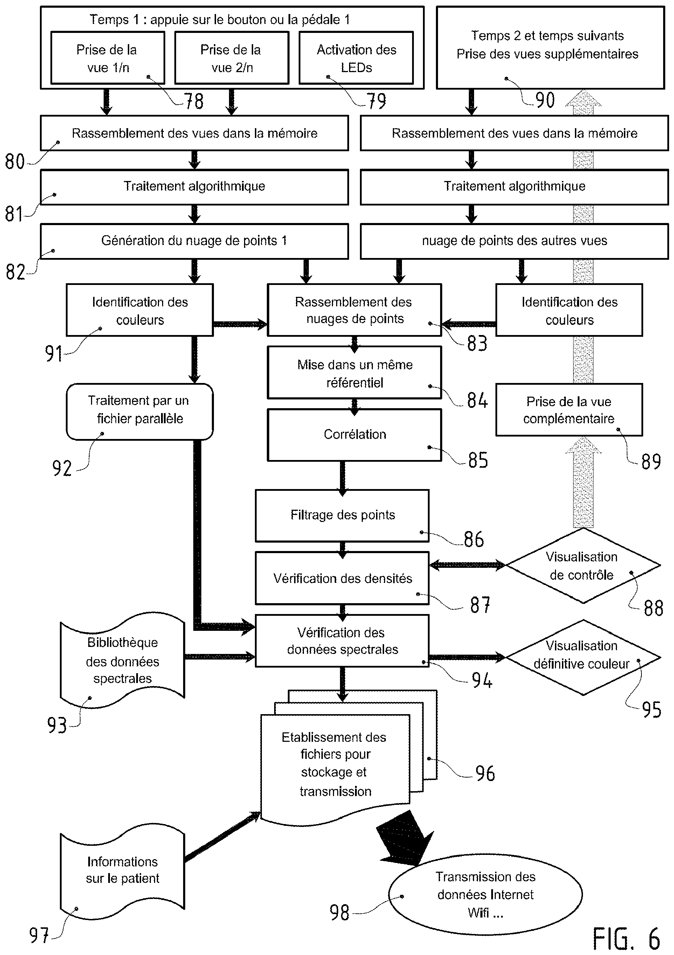

En référence maintenant à la figure 6, on peut voir les différentes étapes du traitement de la mesure dentaire et de l'analyse des tissus utilisant le dispositif, objet de l'invention.

Afin que soit bien comprise la fonction du dispositif objet de la présente invention en dentisterie, ont été présentés dans cette figure 6, les différents états de sa mise en oeuvre. Nous noterons à titre d'exemple qu'il peut exister, et ceci n'est pas limitatif, des temps complémentaires comme l'analyse spectro colorimétrique.

Dans un premier temps, l'opérateur, qu'il soit dentiste au fauteuil, assistant ou technicien de laboratoire, prend la caméra dans la main ce qui a pour effet d'émuler les softwares grâce à un petit cliquet de mise en fonction existant sur le support de la caméra ou inclus dans la caméra elle-même. Il introduit dans la bouche du patient 78, sur l'empreinte ou sur le modèle dupliqué de la vue buccale, la caméra (et visualise sa position à l'aide d'un écran, non visible sur le diagramme mais correspondant à l'écran 5) de la figure 1, la vue que capte la caméra). Il appuie sur le bouton 18 fait démarrer la lecture et l'enregistrement des systèmes de captage des vues en dynamique c'est à dire un film de vues successives et qui ne s'arrêteront qu'au lâché du bouton 18 ou après un deuxième appui. Une fonction de simple lecture sans enregistrement est possible par simple sélection au niveau du menu général sélectionné au moment de la prise en main de la caméra qui pose la question 1: "prise d'empreinte 3, "simple visualisation 3D" ou "simple visualisation 2D" sur l'ordinateur 5.

L'empreinte se complétera d'images en images par le rafraîchissement rapide du capteur et l'envoi des images successives dans la mémoire 80 de la caméra 1 et/ou de l'ordinateur 5 et/ou du boîtier intermédiaire 6. Un traitement algorithmique 81 sera réalisé sur chaque donnée pour en extraire les caractéristiques permettant de connaître la position spatiale et la couleur de chaque point mesuré dans un référentiel. Ceci permettra de générer un nuage de points 82 dans ce référentiel. Ces nuages de points seront rassemblés 83 afin de les mettre dans un référentiel commun 84 puis ils seront corrélés 85 dans un nuage de points unique et de même référentiel. Il va de soi que cette procédure peut être légèrement différente suivant le type de configuration adoptée telles que définies dans les figures 2a à 2e. Les vues étant très riches en points, elles seront filtrées 86 afin d'en extraire les données nécessaires et suffisantes et/ou de les présenter sous forme d'algorithmes ou de simples valeurs matricielles point par point. Il sera possible, par exemple à ce niveau sans que cela s'impose car il est possible de faire cette étape sur une vue vidéo au moment de la prise d'image ou complètement en aval sur les fonctions logarithmiques, de vérifier la densité de l'information en fonction des surfaces mesurées 87. Cette opération est évidemment importante car le bon choix d'un degré de présence d'un nombre de points par unité de surface ou le choix d'un offset judicieux permet de savoir si la mesure est suffisante pour donner une empreinte optique précise. Cette vérification des données se fera 87 et conduira, soit à une validation de l'empreinte soit à un complément de prises de vues 89 et 90. Cette décision peut être prise grâce à une présentation sur l'écran 5 des zones mesurées 88 en couleur rouge (par exemple) pour les zones à compléter et par une visualisation verte (par exemple) pour les zones suffisamment riches.

Les vues complémentaires suivront le même chemin que les vues initiales et viendront compléter, par exemple en 83 le nuage de points insuffisant.

Il ne restera plus qu'à réunir les données validées 94 de chaque point du nuage et de faire, éventuellement, une visualisation définitive 95. Le tout conduira à la conception d'un premier type de fichier dit "fichier de prise d'empreinte optique" 96.

Comme nous l'avons dit, lors de la prise de vue 78 est activé l'éclairage à LEDs 79. Aux questions de choix de types de vue s'ajoute trois autres questions dans le menu "empreinte optique" ou "analyse spectrale" ou "analyse pathologique". Ce deuxième choix permet de définir le type d'éclairage choisi. Si nous optons pour l'empreinte optique, ce seront les LEDs dites "lumière du jour" 14 ou composées de LEDs qui par leurs complémentarités donnent une lumière de spectre connu 33, qui seront activées. L'empreinte sera donc en couleur ce qui permettra de générer l'information demandée 91. Cette information viendra soit compléter le nuage de points 83 soit remplir un fichier spécifique couleur 92 qui, comparé à des fichiers mémorisés 93 permettra une identification grossière de la teinte. Si nous souhaitons mieux connaître la teinte, nous effectuerons un balayage spectrale de l'IR à UV en activant les LEDs spécifiques 60-61-62-64 et successives " en choisissant la fonction "analyse spectrale". Ces deux fichiers permettront de générer un deuxième type de fichier 96 spécifique "couleurs en vues tridimensionnelles" et issu d'une analyse spectro colorimétrique. Enfin en activant la fonction "analyse pathologique" nous activerons spécifiquement ou/et successivement ces mêmes LEDs à une intensité pénétrante propre pour chaque pathologie recherchée ce qui nous permettra de générer un troisième type de fichier dit "pathologique" 96 avec une vue tridimensionnelle colorée montrant les détections effectuées.

Ces fichiers, de premier, deuxième et/ou de troisième type, seront adossés à la fiche du patient (97) pré établie, et transmise localement (Wifi, câbles USB, Ethernet ...) ou extérieurement (Internet ...) sous un format spécifique ou standard (STL ...).

Nous voyons donc, qu'à la différence de tous les systèmes connus, la présente invention permet de générer des fichiers d'empreintes optiques dynamiques en couleur avec des données spectrales correspondant à des mesures de spectro colorimétrie.

Avantageusement selon l'invention, il est possible de suivre les mouvements mandibulaires en plaçant la caméra dans la zone vestibulaire des mâchoires de la bouche. des traits de couleur rouge, et ceci n'est qu'un exemple non limitatif, sonts tracés sur le maxillaire supérieur et sur le maxillaire inférieur puis les mouvements des deux maxillaires sont filmés en vue vestibulaire, du point de départ jusqu'à la fin du mouvement. La caméra prend des vues où un nuage de points se déplace (le maxillaire inférieur) par rapport à l'autre nuage de points (le maxillaire supérieur considéré par principe immobile). Le fait que le marquage appartienne, de manière indépendante, à chacun des maxillaires, le système ne suivra que le déplacement des marquages colorés, mis en valeur au moment de l'éclairement de la LED rouge. Ce même marquage existant au moment de l'empreinte optique faite séparément du maxillaire supérieur et du maxillaire inférieur, le logiciel de corrélation utilisera ce repérage coloré non seulement pour corréler les images de chacun des maxillaires mais aussi pour visualiser les mouvements en fonction de la quatrième dimension, le temps.

Avantageusement, et ceci reste un point très intéressant de l'invention, il est possible de travailler en 2D couleur à partir de vues 3D. Ceci peut se faire de deux manières différentes:

- Comme on utilise une lumière du jour 79, sans projection de trames ou autres lumières structurées, l'écran de visualisation 5 dans notre démarche de contrôle durant les prises de vues 78, nous permet d'utiliser cette caméra d'empreinte optique comme une simple caméra 2D limitant significativement le coût d'investissement des praticiens.
- nous pouvons aussi effectuer cette visualisation 2D, après traitement numérique et mise en évidence des zones pathologiques mises en valeur par le balayage des LEDs de longueurs d'onde spécifiques. Cette technique n'est évidemment possible qu'à partir d'images 3D.

## Revendications

1. Dispositif de prise de vue adapté à prendre en trois dimensions des empreintes optiques d'un objet à étudier dans le domaine dentaire, le dispositif comprenant, d'une part, un système d'éclairage à diodes électroluminescentes (14, 15, 27, 33, 37, 59, 60, 61, 62, 63, 66) adaptées à éclairer l'objet, et, d'autre part, une caméra (1) qui comporte un jeu d'au moins deux capteurs (20) adaptés à capter une image de l'objet, **caractérisé en ce qu'**il comprend un système optique de focalisation (10, 19, 21 53, 64, 65) immobile et à focale fixe qui est adapté à transmettre sans déformation l'image de l'objet aux capteurs (20), la caméra (1) étant une caméra stéréoscopique dont les capteurs (20) sont immobiles, les capteurs (20) de chaque jeu étant placés derrière le système optique de focalisation (10, 19, 21, 53, 64, 65) afin d'observer l'ensemble de l'objet selon un angle différent, de sorte que la caméra (1) est adaptée à prendre l'empreinte optique tridimensionnelle en couleur de l'objet en un seul cliché qui est réalisé simultanément sur l'ensemble des capteurs (20) du jeu, sans avoir à projeter sur l'objet une lumière structurée ou à déposer sur celui-ci une couche blanche.

2. Dispositif de prise de vue selon la revendication 1, **caractérisé en ce qu'**à chaque capteur (20) est associé un système optique de focalisation (10, 19, 21, 53, 64, 65).

3. Dispositif de prise de vue selon l'une des revendications 1 et 2, **caractérisé en ce que** la caméra (1) comprend une tête (7) adaptée à être disposée dans une bouche d'un patient.

4. Dispositif de prise de vue selon la revendication 3, **caractérisé en ce que** la caméra (1) comprend des moyens de transmission d'image (22, 24, 26, 39, 40, 43) qui sont adaptés à transmettre l'image de l'objet au système optique de focalisation (10, 19, 21, 53, 64, 65) disposé hors de la tête (7).

5. Dispositif de prise de vue selon la revendication 4, **caractérisé en ce que** les moyens de transmission d'image comprennent un miroir (22, 24, 39, 40, 43).

6. Dispositif de prise de vue selon l'une des revendications 4 et 5, **caractérisé en ce que** les moyens de transmission d'image comprennent une fibre optique de transmission d'image (26).

7. Dispositif de prise de vue selon l'une des revendications 4 à 6, **caractérisé en ce que** la caméra (1) comprend une fibre optique de transmission de lumière (28) adaptée à transmettre à l'objet la lumière émise par une diode électroluminescente (27) qui est disposée hors de la tête (7).

8. Dispositif de prise de vue selon l'une des revendications 1 à 7, **caractérisé en ce que** les diodes électroluminescentes (14, 15, 27, 33, 37, 59, 60, 61, 62, 63, 66) émettent de la lumière dans différentes longueurs d'onde.

9. Dispositif de prise de vue selon la revendication 8, **caractérisé en ce qu'**il est configuré de façon à permettre l'activation d'une sélection de diodes électroluminescentes (14, 15, 27, 33, 37, 59, 60, 61, 62, 63, 66) permettant d'obtenir un éclairage particulier de l'objet.

10. Dispositif de prise de vue selon l'une des revendications 1 à 9, **caractérisé en ce qu'**il est configuré de façon à prendre des images en deux dimensions de l'objet en n'utilisant qu'un seul capteur (20) par jeu de capteurs.

**11.** Dispositif de prise de vue selon l'une des revendications 1 à 10, **caractérisé en ce que** la caméra (1) comprend, d'une part, une première partie (46) avec le système d'éclairage, les capteurs (20) et le système optique de focalisation (10, 19, 21, 53, 64, 65), et, d'autre part, une seconde partie (47) qui comprend un système de traitement d'images (36) et qui est séparable de la première partie (46).

**12.** Dispositif de prise de vue selon l'une des revendications 1 à 11, **caractérisé en ce que** la caméra (1) comprend plusieurs jeux de capteurs (20) agencés les uns par rapport aux autres de façon à pouvoir prendre en trois dimensions une empreinte dentaire d'au moins une hémi-arcade dentaire en un seul cliché réalisé simultanément sur l'ensemble des capteurs (20) de la caméra (1).

**Patentansprüche**

**1.** Bildaufnahmevorrichtung, geeignet um dreidimensionale optische Abdrücke eines zu untersuchenden Gegenstands im Dentalbereich zu nehmen, wobei die Vorrichtung einerseits ein Beleuchtungssystem mit Leuchtdioden (14, 15, 27, 33, 37, 59, 60, 61, 62, 63, 66), die geeignet sind, den Gegenstand zu beleuchten, und andererseits eine Kamera (1), die einen Satz von mindestens zwei Sensoren (20) umfasst, die geeignet sind, ein Bild des Gegenstands aufzunehmen, umfasst, **dadurch gekennzeichnet, dass** sie ein unbewegliches fokussierendes optisches System (10, 19, 21, 53, 64, 65) mit fester Brennweite umfasst, das geeignet ist, das Bild des Gegenstands ohne Verzerrung an die Sensoren zu übertragen, wobei die Kamera (1) eine stereoskopische Kamera ist, deren Sensoren (20) unbeweglich sind, wobei die Sensoren (20) jedes Satzes hinter dem fokussierenden optischen System (10, 19, 21, 53, 64, 65) angebracht sind, um den gesamten Gegenstand in einem anderen Winkel zu beobachten, so dass die Kamera (1) geeignet ist, den dreidimensionalen optischen Farbenabdruck des Gegenstands in einer einzigen Aufnahme aufzunehmen, die gleichzeitig auf allen Sensoren (20) des Satzes durchgeführt wird, ohne strukturiertes Licht auf den Gegenstand projizieren oder auf diesen letzteren eine weiße Schicht abscheiden zu müssen.

**2.** Bildaufnahmevorrichtung nach Anspruch 1, **dadurch gekennzeichnet, dass** jedem Sensor (20) ein fokussierendes optisches System (10, 19, 21, 53, 64, 65) zugeordnet ist

**3.** Bildaufnahmevorrichtung nach einem der Ansprüche 1 und 2, **dadurch gekennzeichnet, dass** die Kamera (1) einen Kopf (7) umfasst, der geeignet ist, in einem Mund eines Patienten angeordnet zu werden.

**4.** Bildaufnahmevorrichtung nach Anspruch 3, **dadurch gekennzeichnet, dass** die Kamera (1) Bildübertragungsmittel (22, 24, 26, 39, 40, 43) umfasst, die geeignet sind, das Bild des Gegenstands an das außerhalb des Kopfes (7) angeordnete fokussierende optische System (10, 19, 21, 53, 64, 65) zu übertragen.

**5.** Bildaufnahmevorrichtung nach Anspruch 4, dadurch gekennzeichet, dass die Bildübertragungsmittel einen Spiegel (22, 24, 39, 40, 43) umfassen.

**6.** Bildaufnahmevorrichtung nach einem der Ansprüche 4 und 5, **dadurch gekennzeichnet, dass** die Bildübertragungsmittel eine Bildübertragungs-Glasfaser (26) umfassen.

**7.** Bildaufnahmevorrichtung nach einem der Ansprüche 4 bis 6, **dadurch gekennzeichnet, dass** die Kamera (1) eine Lichtübertragungs-Glasfaser (28) umfasst, die geeignet ist, auf den Gegenstand das von einer außerhalb des Kopfes (7) angeordneten Leuchtdiode (27) emittierte Licht zu übertragen.

**8.** Bildaufnahmevorrichtung nach einem der Ansprüche 1 bis 7, **dadurch gekennzeichnet, dass** die Leuchtdioden (14, 15, 27, 33, 37, 59, 60, 61, 62, 63, 66) Licht in verschiedenen Wellenlängen emittieren.

**9.** Bildaufnahmevorrichtung nach Anspruch 8, **dadurch gekennzeichnet, dass** sie konfiguriert ist, um die Aktivierung einer Auswahl von Leuchtdioden (14, 15, 27, 33, 37, 59, 60, 61, 62, 63, 66) zu ermöglichen, die es erlauben, eine besondere Beleuchtung des Gegenstandes zu erhalten.

**10.** Bildaufnahmevorrichtung nach einem der Ansprüche 1 bis 9, **dadurch gekennzeichnet, dass** sie konfiguriert ist, um zweidimensionale Bilder des Gegenstandes unter Benutzung von nur einem Sensor (20) per Sensorensatz zu nehmen.

**11.** Bildaufnahmevorrichtung nach einem der Ansprüche 1 bis 10, **dadurch gekennzeichnet, dass** die Kamera (1) einerseits einen ersten Teil (46) mit dem Beleuchtungssystem, den Sensoren (20) und dem fokussierenden optischen System (10, 19, 21, 53, 64, 65) und andererseits einen zweiten Teil (47) umfasst, der ein Bildverarbeitungssystem (36) umfasst und der von dem ersten Teil (46) trennbar ist.

12. Bildaufnahmevorrichtung nach einem der Ansprüche 1 bis 11, **dadurch gekennzeichnet, dass** die Kamera (1) mehrere Sätze von Sensoren (20) umfasst, die so zu einander angeordnet sind, dass sie bei einer einzigen Aufnahme einen dreidimensionalen Zahnabdruck von mindestens einer Zahnbogenhälfte gleichzeitig auf allen Sensoren (20) der Kamera (1) nehmen können.

**Claims**

1. An image capturing device adapted for capturing three-dimensional optical impressions of an object to be examined in the dental field, the device comprising, on the one hand, a lighting system with light-emitting diodes (14, 15, 27, 33, 37, 59, 60, 61, 62, 63, 66) adapted for illuminating the object and, on the other hand, a camera (1) that includes a set of at least two sensors (20) adapted for capturing an image of the object, wherein it comprises a stationary focusing optical system (10, 19, 21 53, 64, 65) with a fixed focal length that is adapted for transmitting without distortion the image of the object to the sensors (20), the camera (1) being a stereoscopic camera the sensors (20) of which are stationary, the sensors (20) of each set being placed behind the focusing optical system (10, 19, 21, 53 , 64, 65) in order to observe the entire object according to a different angle, so that the camera (1) is adapted for capturing the three-dimensional optical impression in color of the object in a single snapshot that is taken simultaneously on all the sensors (20) of the set, without having to project structured light onto the object or to deposit a white layer thereon.

2. The image capturing device according to claim 1, wherein to each sensor (20) is associated a focusing optical system (10, 19, 21, 53, 64, 65).

3. The image capturing device according to one of claims 1 and 2, wherein the camera (1) comprises a head (7) adapted to be arranged in a patient's mouth.

4. The image capturing device according to claim 3, wherein the camera (1) comprises image transmission means (22, 24, 26, 39, 40, 43) that are adapted for transmitting the image of the object to the focusing optical system (10, 19, 21, 53, 64, 65) arranged outside the head (7).

5. The image capturing device according to claim 4, wherein the image transmission means comprise a mirror (22, 24, 39, 40,43).

6. The image capturing device according to one of claims 4 and 5, wherein the image transmission means comprise an image transmission optical fiber (26).

7. The image capturing device according to one of claims 4 to 6, wherein the camera (1) comprises a light transmission optical fiber (28) adapted for transmitting to the object the light emitted by a light-emitting diode (27) that is arranged outside the head (7).

8. The image capturing device according to one of claims 1 to 7, wherein the light-emitting diodes (14, 15, 27, 33, 37, 59, 60, 61, 62, 63, 66) emit light at different wavelengths.

9. The image capturing device according to claim 8, wherein it is configured so as to permit the activation of a selection of light-emitting diodes (14, 15, 27, 33, 37, 59, 60, 61, 62, 63, 66) that permit to obtain a particular illumination of the object.

10. The image capturing device according to one of claims 1 to 9, wherein it is configured so as to capture two-dimensional images of the object using only one sensor (20) per set of sensors.

11. The image capturing device according to one of claims 1 to 10, wherein the camera (1) comprises, on the one hand, a first portion (46) with the lighting system, the sensors (20) and the focusing optical system (10, 19, 21, 53, 64, 65) and, on the other hand, a second portion (47) that comprises an image-processing system (36) and that is separable from the first portion (46).

12. The image capturing device according to one of claims 1 to 11, wherein the camera (1) comprises several sets of sensors (20) arranged with respect to each other so as to be capable of capturing a three-dimensional dental impression of at least one dental half-arcade in a single snapshot taken simultaneously on all the sensors (20) of the camera (1).

FIG. 1

FIG. 2a

FIG. 2b

FIG. 2c

FIG. 2d

FIG. 2e

FIG. 2f

FIG. 3a

## FIG. 3b

## FIG. 3c

## FIG. 3d

## FIG. 3e

EP 2 579 766 B1

FIG. 4a

Type 1

Type 2

Type 3

24

FIG. 4b

FIG. 4c

FIG. 5a

FIG. 5b

FIG. 5c

Temps 1 : appuie sur le bouton ou la pédale 1

| Prise de la vue 1/n | Prise de la vue 2/n | Activation des LEDs |

Temps 2 et temps suivants
Prise des vues supplémentaires

—78          —79          —90

Rassemblement des vues dans la mémoire

Rassemblement des vues dans la mémoire

80—

Traitement algorithmique

Traitement algorithmique

81—

Génération du nuage de points 1

nuage de points des autres vues

82—

Identification des couleurs

Rassemblement des nuages de points

Identification des couleurs

91—                                    —83

Mise dans un même référentiel

Traitement par un fichier parallèle

—84

92—

Corrélation

Prise de la vue complémentaire

—85          —89

Filtrage des points

—86

Vérification des densités

Visualisation de contrôle

—87          —88

Bibliothèque des données spectrales

Vérification des données spectrales

Visualisation définitive couleur

—94          —95

93—

Etablissement des fichiers pour stockage et transmission

—96

Informations sur le patient

Transmission des données Internet Wifi ...

97—          98—

FIG. 6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4663720 A
- US 4742464 A
- US 4611288 A
- US 5092022 A
- FR 8405173
- US 4952149 A
- WO 9400074 A
- US 7399181 B
- US 10726257 B
- US 7335876 B
- US 7361018 B
- US 7361017 B
- US 7393208 B
- US 6318994 B
- US 6802713 B
- US 11405972 B
- US 7372642 B
- US 20090227875 A
- US 2009259098 D1
- US 2008045789 A
- EP 2166303 A